# EUROPEAN PATENT SPECIFICATION

(11) **EP 3 649 256 B1**
(45) Date of publication and mention of the grant of the patent: **18.02.2026**
(21) Application number: 18829005.0
(22) Date of filing: 04.07.2018
(51) Int. Cl.: C12N 15/113, A61K 31/4174, A61K 31/713, A61P 19/00, A61P 37/00

(54) **METHODS FOR TREATING INFLAMMATION AND RELATED DISEASES AND DISORDERS BY INHIBITING ALPHA PROTEIN KINASE 1**
VERFAHREN ZUR BEHANDLUNG VON ENTZÜNDUNGEN UND VERWANDTEN ERKRANKUNGEN UND ERKRANKUNGEN DURCH HEMMUNG DER ALPHA-PROTEINKINASE 1
PROCÉDÉS DE TRAITEMENT D'UNE INFLAMMATION ET DE MALADIES ET TROUBLES ASSOCIÉS PAR INHIBITION DE LA PROTÉINE KINASE ALPHA 1

(30) Priority: 06.07.2017 WO PCT/CN2017/091995
(43) Date of publication of application: 13.05.2020
(73) Proprietor: Shanghai Yao Yuan Biotechnology Co., Ltd., Shanghai 200437 (CN)
(72) Inventor: XU, Tian, Shanghai 200437 (CN); LI, Tongruei Raymond, Shanghai 200437 (CN); XU, Cong, Shanghai 200437 (CN); FAN, Jieqing, Shanghai 200437 (CN); LIU, Danyang, Shanghai 200437 (CN); PAN, Yanfang, Shanghai 200437 (CN)
(74) Representative: Haseltine Lake Kempner LLP
(86) International application number: PCT/CN2018/094477
(87) International publication number: WO 2019/007362

(56) References cited:
- WO-A1-2015/170108
- WO-A2-2013/023084
- US-A1- 2009 098 056
- US-A1- 2009 098 056
- CHI-PIN LEE ET AL: "ALPK1 phosphorylates myosin IIA modulating TNF-[alpha] trafficking in gout flares", SCIENTIFIC REPORTS, vol. 6, no. 1, 12 May 2016 (2016-05-12), XP55764968, DOI: 10.1038/srep25740
- JULIANE STRIETZ ET AL: "ERN1 and ALPK1 inhibit differentiation of bi-potential tumor-initiating cells in human breast cancer", ONCOTARGET, vol. 7, no. 50, 4 November 2016 (2016-11-04), United States, pages 83278 - 83293, XP55771421, ISSN: 1949-2553, DOI: 10.18632/oncotarget.13086
- MILICA MILIVOJEVIC ET AL: "ALPK1 controls TIFA/TRAF6-dependent innate immunity against heptose-1,7-bisphosphate of gram-negative bacteria", PLOS PATHOGENS, vol. 13, no. 2, 21 February 2017 (2017-02-21), pages e1006224, XP55364679, DOI: 10.1371/journal.ppat.1006224
- ANDRE BLEICH ET AL: "Cdcs1 a major colitis susceptibility locus in mice; Subcongenic analysis reveals genetic complexity", INFLAMMATORY BOWEL DIS, vol. 16, no. 5, 23 October 2009 (2009-10-23), US, pages 765 - 775, XP55771780, ISSN: 1078-0998, DOI: 10.1002/ibd.21146
- YOSHIJI YAMADA ET AL: "Association of genetic variants of the [alpha]-kinase 1 gene with type 2 diabetes mellitus in a longitudinal population-based genetic epidemiological study", BIOMEDICAL REPORTS MAY 2014 SPANDIDOS PUBLICATIONS GBR, vol. 3, no. 3, 2 March 2015 (2015-03-02), Greece, pages 347 - 354, XP55771483, ISSN: 2049-9434, DOI: 10.3892/br.2015.439
- SHU-JUNG WANG ET AL: "Lymphocyte alpha-kinase is a gout-susceptible gene involved in monosodium urate monohydrate-induced inflammatory responses", JOURNAL OF MOLECULAR MEDICINE, SPRINGER, BERLIN, DE, vol. 89, no. 12, 7 August 2011 (2011-08-07), pages 1241 - 1251, XP019979262, ISSN: 1432-1440, DOI: 10.1007/S00109-011-0796-5
- KUO TZER-MIN ET AL: "ALPK1 affects testosterone mediated regulation of proinflammatory cytokines production", JOURNAL OF STEROID BIOCHEMISTRY AND MOLECULAR BIOLOGY, ELSEVIER SCIENCE LTD., OXFORD, GB, vol. 154, 11 August 2015 (2015-08-11), pages 150 - 158, XP029296844, ISSN: 0960-0760, DOI: 10.1016/J.JSBMB.2015.08.007
- LEE MOO-SEUNG ET AL: "Shiga toxins expressed by human pathogenic bacteria induce immune responses in host cells", THE JOURNAL OF MICROBIOLOGY, vol. 51, no. 6, 19 December 2013 (2013-12-19), KR, pages 724 - 730, XP55807451, ISSN: 1225-8873, Retrieved from the Internet <URL:https://link.springer.com/content/pdf/10.1007/s12275-013-3429-6.pdf> DOI: 10.1007/s12275-013-3429-6
- HSIEN-FENG LIAO ET AL: "Down-regulated and Commonly mutated ALPK1 in Lung and Colorectal Cancers", SCIENTIFIC REPORTS, vol. 6, no. 1, 10 June 2016 (2016-06-10), XP055594962, DOI: 10.1038/srep27350

## Description

### FIELD OF THE INVENTION

The present invention relates to an ALPK1 inhibitor for use in a method for treating a disease or disorder caused by a bacterial infection in a subject in need of such treatment, wherein the disease or disorder is sepsis or a cytokine storm, the method comprising administering the ALPK1 inhibitor to the subject, wherein the ALPK1 inhibitor is
(i) an ALPK1 directed antibody or an anti-ALPK1-Fc fusion protein,
(ii) an ALPK1 antisense polynucleotide, or
(iii) an ALPK1-directed interfering RNA selected from the group consisting of a micro RNA (miRNA), a small interfering RNA (siRNA), and short hairpin RNA (shRNA). This disclosure also relates to methods for treating inflammation and related diseases and disorders by inhibiting alpha protein kinase 1 (ALPK1).

### BACKGROUND OF THE INVENTION

The studies on mechanism of inflammatory response have identified various protein kinases that act as essential signaling components. Defects in protein kinase are frequently associated with the pathogenesis of human inflammatory diseases, cancer and diabetes.

Alpha-kinases represent a novel protein kinase superfamily, displaying little sequence similarity to conventional protein kinases. A total of six alpha kinase members including alpha-protein kinase 1 (ALPK1), ALPK2, ALPK3, elongated factor-2 kinase (eEF2K), and transient receptor potential cation channel M6 and M7 (TRPM6 and TRPM7) have been identified. Ryazanov AG et al., Curr Biol 1999, 9(2):R43-45; Ryazanov AG et al., Proc Natl Acad Sci USA 1997, 94(10):4884-4889.

ALPK1 was identified as a new component of raft-containing sucrose-isomerase (SI) vesicles in epithelial cells. Heinet M et al., J. Biol. Chem. (2005) 280(27): 25637-25643. It was shown that ALPK1 phosphorylates myosin 1 and plays an essential role in the exocytic transport to the apical plasma membrane. A transposon-inserted homozygous inactivating mutation of ALPK1 in mice resulted in motor coordination deficits which could be rescued by overexpressing full-length ALPK1. Chen M et al., (2011) BMC Neurosci. 12:1*.*

Several genetic association studies implicated ALPK1 in risk for gout, although not all of the identified polymorphisms replicated in all populations. Wang SJ et al., (2011) J. Mol. Med. 89:1241-1251; Ko AM et al., (2013) J. Intl. Epidemiol. 42: 466-474; Chiba T et al., (2015) Human Cell 28:1-4. Other genetic association studies linked ALPK1 as a risk factor for chronic kidney disease, myocardial infarction, and diabetes. Yamada Y et al. (2013) J Med Genet 50:410-418; Fujimaki T et al., (2014) Biomed Report. 2:127-131; Shimotaka S et al., (2013) Biomed Report. 1:940-944; Yamada Y et al., (2015) Biomed. Report DOI: 10.3892/br.2015.439.

Additional functional studies have implicated ALPK1 as involved in the immune response. For example, ALPK1 was suggested to be a regulator of innate immunity against bacteria through its promotion of TIFA oligomerization and IL-8 expression in response to infection with *S. flexneri, S. typhimurium,* and *Neisseria meningitides.* Milivojevic M et al., (2017) PLoS Pathog 13(2): e1006224. Overexpression of ALPK1 in mice resulted in lower levels of testosterone and increased production of the pro-inflammatory cytokines IL-1β and TGF-β, suggesting that the balance between ALPK1 and testosterone might play a role in testosterone-mediated inhibition of pro-inflammatory cytokines. Kuo TM et al., (2015) J Steroid Biochem Mol Biol (2015) 154: 150-158. Recently, myosin IIA was shown to interact with ALPK1 modulating TNF-trafficking in gout flares. Lee CP et al., (2016) Sci. Report 6:25740.

ALPK1 expression and mutations have also been found in certain cancers, including lung, colorectal, and breast cancers. Liao HF et al. (2016) Scientific Reports. 6:27350; Strietz J et al., (2016) Oncotarget 1-16*.*

In a mouse model of hyperglycemia, overexpression of ALPK1 accelerated multiple early nephropathies. Kuo TM et al., (2016). Biochimka Biophysika Acta 1862:2034-2042.
US 2009/098056 relates to gene variants in diagnosis of gout.
Bleich, A. et al Inflamm. Bowel Dis. vol 16, no. 5 1 May 2010 comments on cytokine-deficiency-induced colitis susceptibility (Cdcs)1 a major colitis susceptibility locus in mice; subcongenic analysis reveals genetic complexity.
Moo-Seung, L. et al J. Microbiol. Vol 51, no 6. 19 Dec 2023, relates to shiga toxins expressed by human pathogenic bacteria induce immune responses in host cells.

There are many diseases, disorders, and conditions whose clinical manifestations result from excessive and/or chronic inflammation. There is a need for new methods for inhibiting and/or reducing inflammation in target tissues for treating such diseases, disorders, and conditions. The present disclosure addresses this need.

### SUMMARY

The present invention is based, in part, on the discovery that inhibition of ALPK1 is effective to reduce inflammation and its clinical effects in several different animal models of inflammation, as well as reducing the production of pro-inflammatory cytokines in diverse human and murine cell lines. Accordingly, the disclosure provides methods of treating inflammation in a subject in need of such treatment, the method comprising administering to the subject an ALPK1 inhibitor. The disclosure also provides methods for treating an inflammatory disease, disorder, or condition, the methods comprising administering an ALPK1 inhibitor to a subject in need of such treatment. An inflammatory disease, disorder, or condition, is characterized by chronic or excessive inflammation.

The present invention is defined by the appended claims. The present invention provides an ALPK1 inhibitor for use in a method for treating a disease or disorder caused by a bacterial infection in a subject in need of such treatment, wherein the disease or disorder is sepsis or a cytokine storm, the method comprising administering the ALPK1 inhibitor to the subject, wherein the ALPK1 inhibitor is
(i) an ALPK1 directed antibody or an anti-ALPK1-Fc fusion protein,
(ii) an ALPK1 antisense polynucleotide, or
(iii) an ALPK1-directed interfering RNA selected from the group consisting of a micro RNA (miRNA), a small interfering RNA (siRNA), and short hairpin RNA (shRNA).

An inflammatory disease or disorder can be selected from inflammatory bowel disease, arthritis, obesity, gout, radiation-induced inflammation, psoriasis, cardiovascular disease, diabetes, epithelial cancers including cancers of the lung, colon, and breast, T cell-mediated hypersensitivity diseases, allergic diseases, atopic dermatitis, non-alcoholic steatohepatitis (NASH), Alzheimer's disease, systemic lupus erythematosus (SLE), autoimmune thyroiditis (Grave's disease), multiple sclerosis, ankylosing spondylitis and bullous diseases due to overproduction of pro-inflammatory cytokines.

An inflammatory disease or disorder can be selected from inflammatory bowel disease, arthritis, obesity, and radiation-induced inflammation. An inflammatory bowel disease can bes selected from Crohn's disease and ulcerative colitis.

An ALPK1 inhibitor can be a kinase inhibitor. An kinase inhibitor is 1-benzyl-3-hexadecyl-2-methyl-1H-imidazol-3-ium iodized salt, or other halogen salts thereof.

In embodiments, the ALPK1 inhibitor is an ALPK1 directed antibody or an anti-ALPK1-Fc fusion protein.

In embodiments, the ALPK1 inhibitor is an ALPK1 antisense polynucleotide. In embodiments, the ALPK1 inhibitor is an ALPK1-directed interfering RNA selected from the group consisting of a micro RNA (miRNA), a small interfering RNA (siRNA), and short hairpin RNA (shRNA).

In embodiments, the disclosure also provides a pharmaceutical composition comprising an ALPK1 inhibitor, and a carrier or excipient, for use in the methods described herein. In embodiments, the ALPK1 inhibitor is a kinase inhibitor. In embodiments, the pharmaceutical composition is formulated for delivery by an oral or rectal route. In embodiments, the pharmaceutical composition is formulated as an oral dosage form in the form of a tablet or capsule. In embodiments, the pharmaceutical composition is formulated as a rectal dosage form in the form of an ointment, suppository, or enema. In embodiments, the pharmaceutical composition is formulated as a parenteral dosage form. In embodiments, the parenteral dosage form is suitable for administration by an intravenous, intra-arterial, or intramuscular route, *e.g.,* by injection of an aqueous liquid.

The disclosure also provides a method for treating an autoimmune disease, a disease or disorder caused by a bacterial infection, or cancer, in a subject in need of such treatment, the method comprising administering to the subject an alpha-kinase 1 (ALPK1) inhibitor.

Where the method is a method of treating cancer, the cancer may be selected from lung, colorectal, and breast cancer.

Where the method is a method for treating an autoimmune disease, the autoimmune disease may be selected from systemic vasculitis, glomerulonephritis (poststreptococcal glomerulonephritis), Sjogren's syndrome, psoriatic arthritis, gout, gouty arthritis, reactive arthritis, septic shock, Graves' disease, Goodpasture syndrome, myasthenia gravis, autoimmune hemolytic anemia, idiopathic thrombocytopenic purpura, autoimmune myositis, pernicious anemia, celiac disease, eczema, autoimmune thyroiditis, autoimmune myocarditis, celiac disease, juvenile idiopathic arthritis, Graves ophthalmopathy, polymyalgia rheumatica, autoimmune uveoitis, alopecia areata, wegener, vitiligo, primary sclerosing cholangitis, primary biliary cirrhosis, autoimmune hepatitis, Guillain-Barré syndrome, antiphospholipid syndrome, sarcoidosis pain, alopecia areata, Lambert-Eaton myasthenic syndrome, autoimmune hemolytic anemia, cold agglutinin disease, warm autoimmune hemolytic anemia, eosinophilic granulomatosis with polyangiitis (Churg-Strauss syndrome), and Behcet's disease.

In embodiments where the ALPK1 inhibitor is for use in a method for treating a disease or disorder caused by a bacterial infection, the disease or disorder may be selected from sepsis, and a cytokine storm. In embodiments, the disease or disorder may be caused by a bacteria selected from *Neisseria, Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia and Brucella.*

### BRIEF DESCRIPTION OF THE FIGURES

Fig. 1: Inactivating ALPK1 mutation confers resistance to DSS-induced colitis. Body weight percentage change (%), triangles, wild type (WT); diamonds, heterozygous mutants (HE); squares, homozygous mutants (HO).
Fig. 2A-B: Inactivating ALPK1 mutation confers resistance to radiation induced weight loss, **A** (y-axis shows percentage weight change), and lethality, **B** (y-axis shows survival.
Fig. 3: Inactivating ALPK1 mutation confers resistance to high-fat diet induced obesity. "HFD" refers to "high fat diet treated", y-axis shows percentage weight change.
Fig. 4A-B: Inactivating ALPK1 mutation confers resistance to collagen-induced arthritis. **A,** ALPK1 mutant male footpad RA score. **B,** ALPK1 mutant male left-right hind footpad thickness difference.
Fig. 5: Knockdown of *ALPK1* by siRNA leads to decreased cytokine expression in HEK293T cells.
Fig. 6A-B: A, Knockdown of *ALPK1* in HEK293T cells leads to decreased expression of cytokines upon *S. flexneri* cell lysate induction and overexpression of human and mouse *ALPK1.* **B,** IL8 expression is measured to show efficient IL8 expression by Flag-ALPK1, mouse ALPK1, 6*His- ALPK1-3*Flag, 6*His- ALPK1, but not by the 6*His-ALPK1-E mutant. *ALPK1* can rescue impaired cytokine expression, but not for the kinase dead mutant.
Fig. 7A-B: CRISPR/Cas9 knockout of ALPK1 in HEK293T cells leads to decreased cytokine expression and secretion upon *S. flexneri* cell lysate induction. **A,** Relative *expression* normalized to wild-type HEK293T control. **B,** IL8 concentration in conditioned media of HEK293T 4 hours post *S. flexneri* cell lysate induction.
Fig. 8: Knockdown of *ALPK1* in HEK293 cells leads to decreased expression of cytokines upon *S. flexneri* and *S. typhimurium* cell lysate induction.
Fig. 9A-B: *ALPK1* knockdown by a short hairpin RNA (shRNA) in THP-1 macrophage leads to reduced expression of pro-inflammatory cytokines with LPS induction (A) or without LPS induction **(B).**
Fig. 10A-B: Knockdown of ALPK1 by shRNA in THP-1 macrophage cells results in inhibition of secretion of TNFα **(A)** and IL1β **(B).**
Fig. 11: *ALPK1* knockdown by siRNA in a MDA-MB-468 human breast cancer cell line macrophages resulted in decreased expression of TNFα, IL1β and IL8.
Fig. 12A-B: *ALPK1* knockdown by shRNA in a RAW 264.7 murine macrophage-like cell line leads to decreased cytokine expression either in the presence **(A)** or absence **(B)** of LPS.
Fig. 13: Pro-inflammatory cytokine expression was decreased in bone marrow derived macrophage cells isolated from mice carrying inactivating ALPK1 mutations.
Fig. 14: ALPK1 inhibitor MI6C improves recovery in DSS-induced colitis model. Mice were treated with 3% DSS from day 0 to day 5 then changed to drinking water and injected IP with either MI6C (1 mg/kg) dissolved in DMSO (n=6) or DMSO alone (n=5) daily. Y-axis shows change in weight relative to day 0, X-axis shows time (days).
Fig. 15A-D: ALPK1 mutation reduces tumor growth and metastasis in the MMTV-PyVT breast cancer model. In each panel, mice carrying the ALPK1 mutation are indicated by dark bars and mock transgenic controls by light bars. **A,** breast tumor appearance age; **B,** Breast tumor load at 10 weeks after breast tumor appearance; **C,** lung tumor number at 10 weeks after breast tumor appearance (metastasis from breast tumor); **D,** lung weight at 10 weeks after breast tumor onset (metastasis from breast tumor).
Fig. 16: Amino acid sequence of human ALPK1 isoform 1. The sequence of isoform 2 differs from isoform 1 as follows:
   1-92: MNNQKVVAVL...VIGAGLQQLL → MCRKRTRARTSAAE

### DETAILED DESCRIPTION

The present invention is based, in part, on the discovery that ALPK1 is a potent inducer of the inflammatory response in diverse cell and animal models of inflammation such that its inhibition is effective to inhibit inflammation and ameliorate its deleterious effects. Accordingly, the disclosure provides methods for treating inflammation and for treating diseases, disorders, and conditions characterized by excessive and/or chronic inflammation, as well as methods for treating autoimmune diseases, diseases and disorders caused by bacterial infection, and cancer, by administering to a subject in need of such treatment an ALPK1 inhibitor, or by inhibiting ALPK1 in the subject, for example using a gene therapy approach, and related compositions. The present invention provides an ALPK1 inhibitor for use in a method for treating a disease or disorder caused by a bacterial infection in a subject in need of such treatment, wherein the disease or disorder is sepsis or a cytokine storm, the method comprising administering the ALPK1 inhibitor to the subject, wherein the ALPK1 inhibitor is
(i) an ALPK1 directed antibody or an anti-ALPK1-Fc fusion protein,
(ii) an ALPK1 antisense polynucleotide, or
(iii) an ALPK1-directed interfering RNA selected from the group consisting of a micro RNA (miRNA), a small interfering RNA (siRNA), and short hairpin RNA (shRNA).

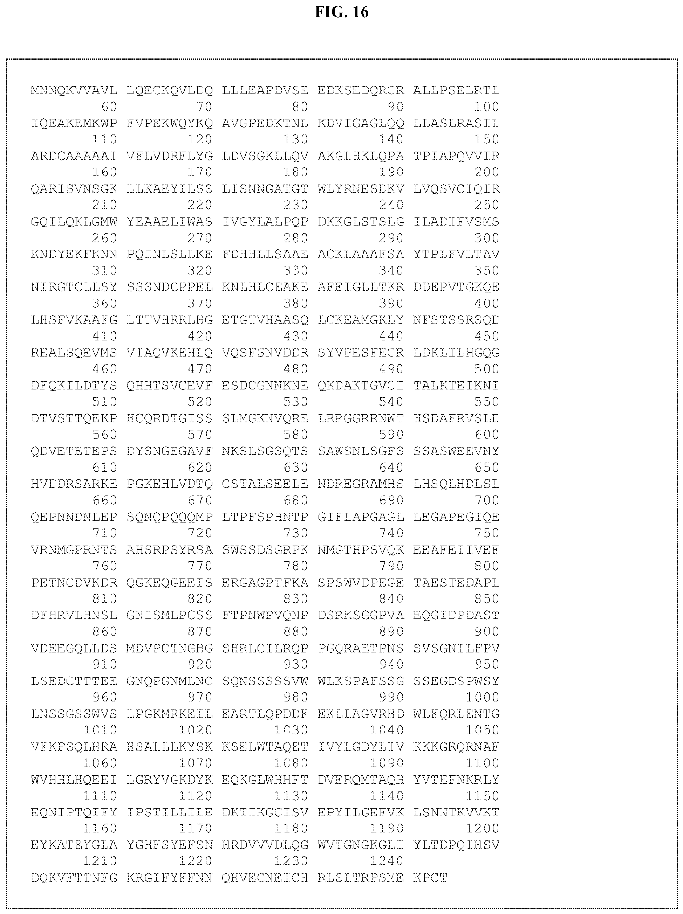

The term "ALPK1" is used herein to refer interchangeably to isoform 1 (Q96QP1-1) or the alternative splice variant isoform 2 (Q96QP1-2) of the human sequence identified by UniProtKB - Q96QP1 (ALPK1_HUMAN), unless the text explicitly refers to a particular isoform. *See* Fig. 16 and SEQ ID NO: 1. Isoform 2 differs from isoform 1 as follows:

### 1-92: MNNQKVVAVL...VIGAGLQQLL → MCRKRTRARTSAAE

An inflammatory disease or disorder can be selected from inflammatory bowel disease, arthritis, obesity, gout, radiation-induced inflammation, psoriasis, cardiovascular disease, diabetes, epithelial cancers including cancers of the lung, colon, and breast, T cell-mediated hypersensitivity diseases, allergic diseases, and atopic dermatitis due to overproduction of pro-inflammatory cytokines.

An inflammatory disease or disorder can be selected from inflammatory bowel disease, arthritis, obesity, and radiation-induced inflammation. In embodiments, the inflammatory bowel disease is selected from Crohn's disease and ulcerative colitis.

An autoimmune disease can be selected from systemic vasculitis, glomerulonephritis (poststreptococcal glomerulonephritis), Sjogren's syndrome, psoriatic arthritis, gout, gouty arthritis, reactive arthritis, septic shock, Graves' disease, Goodpasture syndrome, myasthenia gravis, autoimmune hemolytic anemia, idiopathic thrombocytopenic purpura, autoimmune myositis, pernicious anemia, celiac disease, eczema, autoimmune thyroiditis, autoimmune myocarditis, celiac disease, juvenile idiopathic arthritis, Graves ophthalmopathy, polymyalgia rheumatica, autoimmune uveoitis, alopecia areata, wegener, vitiligo, primary sclerosing cholangitis, primary biliary cirrhosis, autoimmune hepatitis, Guillain-Barré syndrome, antiphospholipid syndrome, sarcoidosis pain, alopecia areata, Lambert-Eaton myasthenic syndrome, autoimmune hemolytic anemia, cold agglutinin disease, warm autoimmune hemolytic anemia, eosinophilic granulomatosis with polyangiitis (Churg-Strauss syndrome), and Behcet's disease.

In embodiments, the disease or disorder caused by a bacterial infection is selected from sepsis, and a cytokine storm. In embodiments, the disease or disorder is caused by a bacteria selected from *Neisseria, Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia and Brucella.*

A cancer can be selected from soft tissue sarcoma, breast cancer, head and neck cancer, melanoma, cervical cancer, bladder cancer, hematologic malignancy, glioblastoma, pancreatic cancer, prostate cancer, colon cancer, breast cancer, renal cancer, lung cancer, Merkel cell carcinoma, small intestine cancer, thyroid cancer, acute myelogenous leukemia (AML), acute lymphocytic leukemia (ALL), chronic lymphocytic leukemia (CLL), chronic myelogenous leukemia (CML), gastric cancer, gastrointestinal stromal tumors, non-Hodgkins lymphoma, Hodgkins lymphoma, liver cancer, leukemia, lymphoma, T-cell lymphoma, brain cancer, and multiple myeloma.

An ALPK1 inhibitor can be is a kinase inhibitor. In one embodiment, the kinase inhibitor is 1-benzyl-3-hexadecyl-2-methyl-1H-imidazol-3-ium (also known as NH-125, CAS 278603-08-0, which may be referred to herein as MI6 or MI6C):

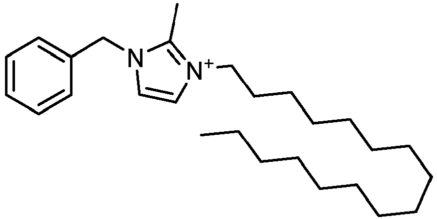

In embodiments, the ALPK1 inhibitor is a halogen salt of 1-benzyl-3-hexadecyl-2-methyl-1H-imidazol-3-ium. In embodiments, the halogen is selected from fluorine, chlorine, bromine, iodine, and astatine. In embodiments, the halogen is iodine.

In embodiments, the ALPK1 inhibitor is an ALPK1 directed antibody or an anti-ALPK1-Fc fusion protein. In embodiments, the antibody is a fully human antibody, a humanized antibody, a camelid antibody, a chimeric antibody, a CDR-grafted antibody, a single-chain Fvs (scFv), a disulfide-linked Fvs (sdFv), an Fab fragment, or an antigen-binding fragment of any of the foregoing. The general term 'antibody' includes immunoglobulin molecules and antigen-binding active fragments thereof, *i.e.,* molecules that contain an antigen binding site. Such fragments may or may not be fused to another immunoglobulin domain including, but not limited to, an Fc region or fragment thereof. Antigen-binding fragments include, for example, Fab, Fab', F(ab')₂ and Fv fragments. These fragments lack the heavy chain constant fragment (Fc) of an intact antibody and are sometimes preferred because they tend to clear more rapidly from the circulation and have less non-specific binding than an intact antibody. Such fragments are produced from intact antibodies using methods known in the art, for example by proteolytic cleavage with enzymes such as papain (to produce Fab fragments) or pepsin (to produce F(ab')₂ fragments). Preferably, an antigen-binding fragment is a dimer of heavy chains (a camelid antibody), a single-chain Fvs (scFv), a disulfide-linked Fvs (sdFv), a Fab fragment, or a F(ab') fragment. The skilled person will appreciate that other fusion products may be generated, including but not limited to, scFv-Fc fusions, variable region (e.g., VL and VH)-Fc fusions, and scFv-scFv-Fc fusions. Immunoglobulin molecules can be of any type, including, IgG, IgE, IgM, IgD, IgA and IgY, and of any class, including IgG₁, IgG₂, IgG₃, IgG₄, IgA₁ and IgA₂), or of any subclass.

Preferably, an antibody for therapeutic use in the methods described here is a monoclonal antibody, preferably an IgG antibody. A monoclonal antibody is derived from a substantially homogeneous population of antibodies specific to a particular antigen, which population contains substantially similar epitope binding sites. Such antibodies may be of any immunoglobulin class including IgG, IgM, IgE, IgA, and any subclass thereof. Methods for monoclonal antibody production are known in the art, e.g., hybridoma technology. In embodiments, the antibody is a chimeric, human, or humanized antibody, or an antigen-binding fragment thereof, preferably which exhibits low toxicity when administered to a subject, preferably a human subject.

In embodiments, the ALPK1 inhibitor is an ALPK1 antisense polynucleotide. In embodiments, the ALPK1 inhibitor is an ALPK1-directed interfering RNA selected from the group consisting of a micro RNA (miRNA), a small interfering RNA (siRNA), and short hairpin RNA (shRNA).

A gene therapy approach may be used to inhibit ALPK1. In embodiments, the gene therapy approach may comprise introducing an inactivating mutation in ALPK1 using a gene editing technique. In embodiments, the gene editing technique may include those based on meganucleases, zinc finger nucleases (ZFNs), transcription activator-like effector nucleases (TALENs), and CRISPR/Cas-9.

In the context of the methods described here, the term "treating" may refer to the amelioration or stabilization of one or more symptoms associated with the disease, disorder or condition being treated. The term "treating" may also encompass the management of disease, disorder or condition, referring to the beneficial effects that a subject derives from a therapy but which does not result in a cure of the underlying disease, disorder, or condition. In the context of the present disclosure, the term "prevention" refers to preventing the recurrence, development, progression or onset of one or more symptoms of the disease, disorder, or condition.

In embodiments where a therapeutically effective amount of a composition is administered to a subject, the therapeutically effective amount is the amount sufficient to achieve a desired therapeutic outcome, for example the amelioration or stabilization of one or more symptoms of the disease, disorder or condition being treated, or in the context of prevention, the amount sufficient to achieve prevention of the recurrence, development, progression or onset of one or more symptoms of the disease, disorder, or condition.

In embodiments, a therapeutically effective amount is the amount required to achieve at least an equivalent therapeutic effect compared to a standard therapy. An example of a standard therapy is an FDA-approved drug indicated for treating the same disease, disorder or condition.

In the context of any of the methods described here, the subject is preferably a human but may be a non-human mammal, preferably a non-human primate. In other embodiments, the non-human mammal may be, for example, a dog, cat, a rodent (*e.g.,* a mouse, a rat, a rabbit), a horse, a cow, a sheep, a goat, or any other non-human mammal.

In embodiments, the human subject is selected from an adult human, a pediatric human, or a geriatric human, as those terms are understood by the medical practitioner, for example as defined by the U.S. Food and Drug Administration.

This disclosure provides an ALPK1 inhibitor in the form of a small organic molecule (*e.g.,* 1-benzyl-3-hexadecyl-2-methyl-1H-imidazol-3-ium iodized salt or other halogen salt thereof). In embodiments an ALPK1 inhibitor is in the form of a large biomolecule such as a protein (*e.g.,* an ALPK1-directed antibody or Fc fragment thereof) or a nucleic acid (*e.g.,* an ALPK1-directed antisense polynucleotide, or interfering RNA such as a micro RNA (miRNA), a small interfering RNA (siRNA), or short hairpin RNA (shRNA). In the context of the present disclosure, the generic term "compound" is meant to encompass both small organic molecules and large biomolecules.

In embodiments, the disclosure provides a composition comprising an ALPK1 inhibitor, and one or more excipients or carriers, preferably pharmaceutically acceptable excipients or carriers. As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Excipients for preparing a pharmaceutical composition are generally those that are known to be safe and non-toxic when administered to a human or animal body. Examples of pharmaceutically acceptable excipients include, without limitation, sterile liquids, water, buffered saline, ethanol, polyol (for example, glycerol, propylene glycol, liquid polyethylene glycol and the like), oils, detergents, suspending agents, carbohydrates (*e.g.,* glucose, lactose, sucrose or dextran), antioxidants (*e.g.,* ascorbic acid or glutathione), chelating agents, low molecular weight proteins, and suitable mixtures of any of the foregoing. The particular excipients utilized in a composition will depend upon various factors, including chemical stability and solubility of the compound being formulated and the intended route of administration.

A pharmaceutical composition can be provided in bulk or unit dosage form. It is especially advantageous to formulate pharmaceutical compositions in unit dosage form for ease of administration and uniformity of dosage. The term "unit dosage form" refers to physically discrete units suited as unitary dosages for the subject to be treated; each unit containing a predetermined quantity of an active compound calculated to produce the desired therapeutic effect in association with the required pharmaceutical carrier. A unit dosage form can be an ampoule, a vial, a suppository, a dragee, a tablet, a capsule, an IV bag, or a single pump on an aerosol inhaler.

In therapeutic applications, dose may vary depending on the chemical and physical properties of the active compound as well as clinical characteristics of the subject, including e.g., age, weight, and co-morbidities. Generally, the dose should be a therapeutically effective amount. An effective amount of a pharmaceutical composition is that which provides an objectively identifiable improvement as noted by the clinician or other qualified observer. For example, alleviating a symptom of a disorder, disease or condition.

A pharmaceutical compositions may take any suitable form (*e.g*. liquids, aerosols, solutions, inhalants, mists, sprays; or solids, powders, ointments, pastes, creams, lotions, gels, patches and the like) for administration by any desired route (*e.g*. pulmonary, inhalation, intranasal, oral, buccal, sublingual, parenteral, subcutaneous, intravenous, intramuscular, intraperitoneal, intrapleural, intrathecal, transdermal, transmucosal, rectal, and the like). In embodiments, the pharmaceutical composition is in the form of an orally acceptable dosage form including, but not limited to, capsules, tablets, buccal forms, troches, lozenges, and oral liquids in the form of emulsions, aqueous suspensions, dispersions or solutions. Capsules may contain excipients such as inert fillers and/or diluents including starches (*e.g.,* corn, potato or tapioca starch), sugars, artificial sweetening agents, powdered celluloses, such as crystalline and microcrystalline celluloses, flours, gelatins, gums, *etc.* In the case of tablets for oral use, carriers which are commonly used include lactose and corn starch. Lubricating agents, such as magnesium stearate, can also be added.

In embodiments, the pharmaceutical composition is in the form of a tablet. The tablet can comprise a unit dose of a compound described here together with an inert diluent or carrier such as a sugar or sugar alcohol, for example lactose, sucrose, sorbitol or mannitol. The tablet can further comprise a non-sugar derived diluent such as sodium carbonate, calcium phosphate, calcium carbonate, or a cellulose or derivative thereof such as methyl cellulose, ethyl cellulose, hydroxypropyl methyl cellulose, and starches such as corn starch. The tablet can further comprise binding and granulating agents such as polyvinylpyrrolidone, disintegrants (*e.g*. swellable crosslinked polymers such as crosslinked carboxymethylcellulose), lubricating agents (*e.g.* stearates), preservatives (*e.g.* parabens), antioxidants (*e.g.* butylated hydroxytoluene), buffering agents (*e.g.* phosphate or citrate buffers), and effervescent agents such as citrate/bicarbonate mixtures. The tablet may be a coated tablet. The coating can be a protective film coating (*e.g.* a wax or varnish) or a coating designed to control the release of the active compound, for example a delayed release (release of the active after a predetermined lag time following ingestion) or release at a particular location in the gastrointestinal tract. The latter can be achieved, for example, using enteric film coatings such as those sold under the brand name Eudragit^{®}.

Tablet formulations may be made by conventional compression, wet granulation or dry granulation methods and utilize pharmaceutically acceptable diluents, binding agents, lubricants, disintegrants, surface modifying agents (including surfactants), suspending or stabilizing agents, including, but not limited to, magnesium stearate, stearic acid, talc, sodium lauryl sulfate, microcrystalline cellulose, carboxymethylcellulose calcium, polyvinylpyrrolidone, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, dry starches and powdered sugar. Preferred surface modifying agents include nonionic and anionic surface modifying agents. Representative examples of surface modifying agents include, but are not limited to, poloxamer 188, benzalkonium chloride, calcium stearate, cetostearyl alcohol, cetomacrogol emulsifying wax, sorbitan esters, colloidal silicon dioxide, phosphates, sodium dodecyl sulfate, magnesium aluminum silicate, and triethanolamine.

In embodiments, the pharmaceutical composition is in the form of a hard or soft gelatin capsule. In accordance with this formulation, the compound for use according to the present invention may be in a solid, semi-solid, or liquid form.

In embodiments, the pharmaceutical composition is in the form of a sterile aqueous solution or dispersion suitable for parenteral administration. The term parenteral as used herein includes subcutaneous, intracutaneous, intravenous, intramuscular, intra-articular, intraarterial, intrasynovial, intrasternal, intrathecal, intralesional and intracranial injection or infusion techniques.

In embodiments, the pharmaceutical composition is in the form of a sterile aqueous solution or dispersion suitable for administration by either direct injection or by addition to sterile infusion fluids for intravenous infusion, and comprises a solvent or dispersion medium containing, water, ethanol, a polyol (*e.g.,* glycerol, propylene glycol and liquid polyethylene glycol), suitable mixtures thereof, or one or more vegetable oils. Solutions or suspensions can be prepared in water with the aid of co-solvent or a surfactant. Examples of suitable surfactants include polyethylene glycol (PEG)-fatty acids and PEG-fatty acid mono and diesters, PEG glycerol esters, alcohol-oil transesterification products, polyglyceryl fatty acids, propylene glycol fatty acid esters, sterol and sterol derivatives, polyethylene glycol sorbitan fatty acid esters, polyethylene glycol alkyl ethers, sugar and its derivatives, polyethylene glycol alkyl phenols, polyoxyethylene-polyoxypropylene (POE-POP) block copolymers, sorbitan fatty acid esters, ionic surfactants, fat-soluble vitamins and their salts, water-soluble vitamins and their amphiphilic derivatives, amino acids and their salts, and organic acids and their esters and anhydrides. Dispersions can also be prepared, for example, in glycerol, liquid polyethylene glycols and mixtures of the same in oils.

In embodiments, a compound or composition described here may be administered as monotherapy or adjunctive therapy. In embodiments, a compound or composition described here may be administered alone or in combination with one or more additional therapeutic agents (*i.e.,* additional APIs) or therapies, for example as part of a therapeutic regimen that includes, *e.g.,* aspects of diet and exercise). In embodiments, the methods described here include administration of an ALPK1 inhibitor as the primary therapy. In other embodiments, the administration of an ALPK1 inhibitor is an adjuvant therapy. In either case, the methods described herein contemplate the administration of an ALPK1 inhibitor in combination with one or more additional therapeutic agents and/or therapies for the treatment or prevention of a disease, disorder, or condition as described here. The terms "therapy" and "therapies" refer to any method, protocol and/or agent that can be used in the prevention, treatment, management or amelioration of a disease, disorder, or condition, one or more symptoms thereof.

The present disclosure also provides packaging and kits comprising pharmaceutical compositions for use in the methods described here. The kit can comprise one or more containers selected from the group consisting of a bottle, a vial, an ampoule, a blister pack, and a syringe. The kit can further include one or more of instructions for use, one or more syringes, one or more applicators, or a sterile solution suitable for reconstituting a compound or composition described here.

All percentages and ratios used herein, unless otherwise indicated, are by weight.

The invention is further described and exemplified by the following nonlimiting examples.

### EXAMPLES

The following examples demonstrate that inhibiting ALPK1 is effective to treat inflammation directly or indirectly.

### Example 1: Inactivating ALPK1 mutation confers resistance to DSS-induced colitis

The murine model of dextran sodium sulfate (DSS)-induced colitis was used to evaluate whether inhibition of ALPK1 *via* an inactivating mutation (a CRISPR vector targeting two sites in the introns, one before exon 13 and one after exon 13, was injected into fertilized mouse embryos to create an ALPK1 mouse mutant with exon 13 deleted. Deletion of exon 13, which encodes one part of kinase domain of ALPK1, kills ALPK's kinase activity) could ameliorate inflammatory bowel disease, such as ulcerative colitis and Crohn's disease. DSS treatment causes breakdown of colon epithelial cells and induces colitis, which in turn causes weight loss. Weight loss was used as the indicator of disease progression. This model system has been described previously, for example in Okayasu H *et al.,* 1990. Briefly, 8-9 week old female mice were treated with 2% DSS (MW 40K-50K, MP BIOANALYTICAL) dissolved in autoclaved drinking water for 7 days. Mice were weighed daily and at the conclusion of the test period. All data were expressed as the mean ± standard error of the mean (SEM). Experimental groups were ALPK1 Wild type (WT) (n=3); heterozygous (HE) inactivating ALPK1 mutation (n=2); and homozygous (HO) inactivating ALPK1 mutation (n=3).

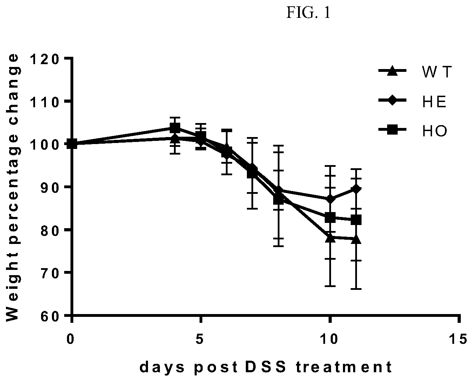

Results: The inactivating ALPK1 mutation showed a protective effect in all three measures of disease progression/severity in the DSS-induced colitis model, weight loss (Fig. 1).

### Example 2: Inactivating ALPK1 mutation confers resistance to radiation-induced Inflammation

A murine model of radiation-induced inflammation was used to evaluate whether inhibition of ALPK1 *via* an inactivating mutation could ameliorate radiation-induced inflammation. Radiation stimulates the immune system, activating an inflammatory response. This model system has been described previously, for example in Biju G *et al.,* 2012. Briefly, 8-9 week old female mice were irradiated to 9 Gray (Gy) total body irradiation (gamma ray from Co60) per mouse once on day 1. Animal survival and weight were measured daily. Radiation caused damage of the gastrointestinal and hematopoietic systems. Translocation of intestinal microflora combined with immune system compromise may lead to septicemia and death.

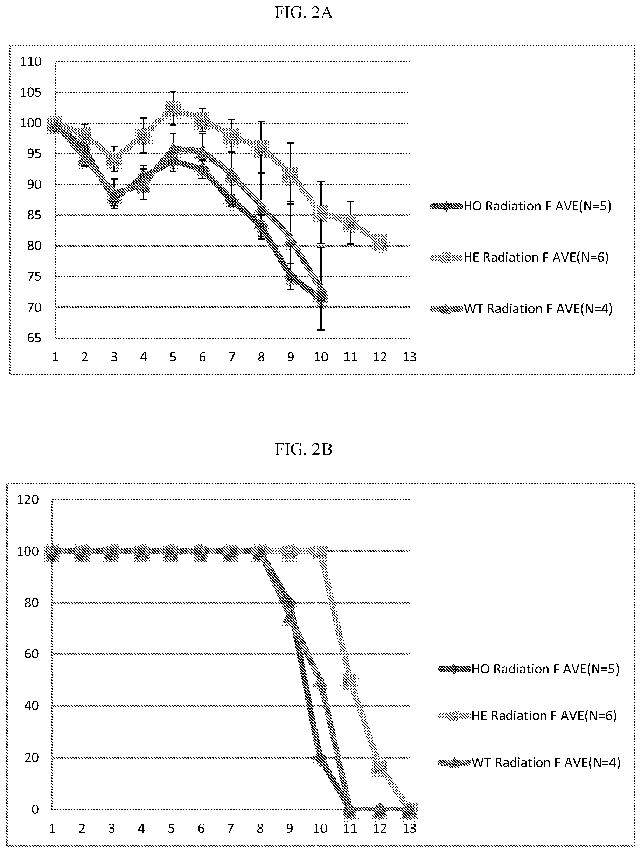

Experimental groups were ALPK1 Wild type (WT) (n=4); heterozygous (HE) inactivating ALPK1 mutation (n=6); and homozygous (HO) inactivating ALPK1 mutation (n=5). In this study, only the heterozygous mutation showed a protective effect over radiation induced weight loss (Fig. 2A) and lethality (Fig. 2B).

### Example 3: Inactivating ALPK1 mutation confers resistance to high fat diet induced obesity

A murine model of high fat diet induced obesity was used to evaluate whether inhibition of ALPK1 *via* an inactivating mutation could ameliorate inflammation in this model system. Obesity shares with many chronic diseases the presence of an inflammatory component that accounts for the development of metabolic disease. This inflammatory state is reflected in increased circulating levels of pro-inflammatory proteins. High fact diet (HFD) feeding can induce obesity and metabolic disorders in rodents that resemble the human metabolic syndrome. This model system has been described previously, for example in Bourgeois A *et al.,* 1983; Takahashi I et al., 1999. Briefly, 8-9 week old male mice were treated with HFD (60% fat, Research Diets Inc., New Brunswick, NJ) from day 0. Body weight was measured weekly from day 1 to day 84 post-HFD. Percentage body weight gain (%g) was expressed as a mean ± standard deviations (SD).

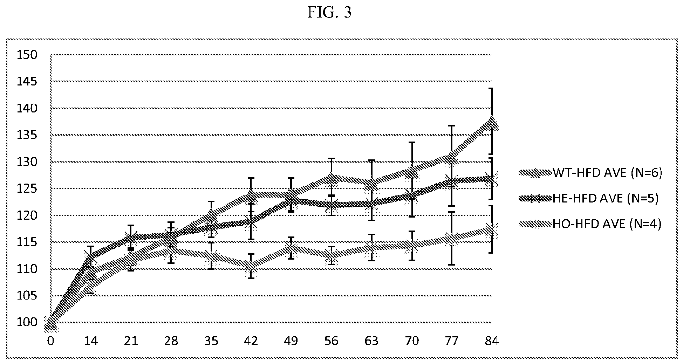

Experimental groups were ALPK1 Wild type (WT) (n=6); heterozygous (HE) inactivating ALPK1 mutation (n=5); and homozygous (HO) inactivating ALPK1 mutation (n=4). In this study, both heterozygous and homozygous inactivating mutations in ALPK1 conferred protection against HFD-induced weight gain (Fig. 3).

### Example 4: Inactivating ALPK1 mutation confers resistance to collagen-induced arthritis

A murine model of collagen-induced arthritis was used to evaluate whether inhibition of ALPK1 *via* an inactivating mutation could ameliorate inflammation in this model system. Rheumatoid arthritis is recognized as an inflammatory immune process, where the body's oversensitive immune system attacks the body's own tissues such as the joint linings and cartilage. The inflammation can cause the pain and swelling common with rheumatoid arthritis. This model system has been described previously, for example in Campbell H et al., 2000. Briefly, 8 week old male mice were treated with 20 µl Bovine Type II Collagen (Immunization Grade Bovine Type II Collagen, Solution (Chondrex, Cat.no. 20022) and a Complete Freund's Adjuvant (CFA, inactivated *Mycobacterium tuberculosis* H37Ra at 4 mg/ml (Chondrex, Cat. no. 7001)) (1 mg/ml final concentration for both) through subdermal injection on the footpad of one hind foot, with the other foot injected with 20 µl PBS control of control. Bovine type II collagen induces a swollen foot due to inflammation. Mice were evaluated for rheumatoid arthritis score and foot thickness for paw swelling was measured every other day up to 25 days post-collagen injection. Rheumatoid arthritis (RA) score is the total of all four paw scores on scale of 0-16, where each paw is scored as follows:
Score 0 - Normal paw;
Score 1 - One toe inflamed and swollen;
Score 2 - More than one toe, but not entire paw inflamed and swollen, or mild swelling of entire paw;
Score 3 - Entire paw inflamed and swollen; and
Score 4 - Very inflamed and swollen or ankylosed (stiffing or immobile) paw.

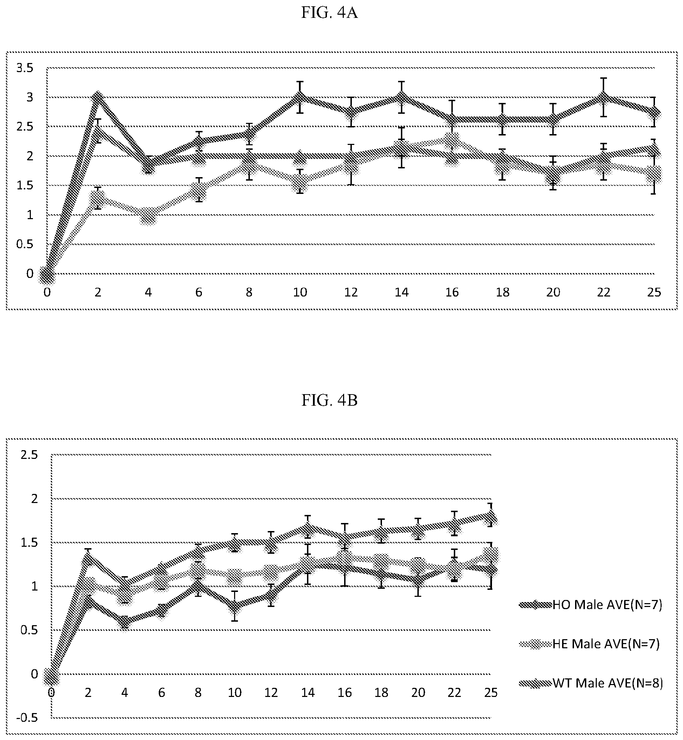

Data are expressed as mean ± standard deviation of number of experimental group as indicated. Experimental groups were ALPK1 Wild type (WT) (n=8); heterozygous (HE) inactivating ALPK1 mutation (n=7); and homozygous (HO) inactivating ALPK1 mutation (n=7). In this study, both indicators of inflammation, the footpad RA score (Fig. 4A) and the footpad thickness difference (Fig. 4B) were lower in mice having heterozygous or homozygous inactivating mutations in ALPK1.

### Example 5: ALPK1 Knockdown inhibits expression of pro-inflammatory cytokines in various human and mouse cells

The results are summarized in Table 1 and described in more detail below.

**Table 1: Downregulated cytokines in the ALPK1 knockdown human and murine cells.**

| **Cell line** | **Human** | **Cell type** | **Downregulated cytokine genes** |
|---|---|---|---|
| HEK293T | Human | Adrenal cell | IL8, IL10, TNFα |
| HEK293 | Human | Adrenal cell | IL8, IL10, TNFα |
| HeLa | Human | Cervix epithelium | IL1β, IL6, IL8, TNFα, IFNγ |
| THP1-macrophage | Human | Macrophage | IL1β, IL8, IL10, TNFα |
| MDA-MB-468 | Human | Mammary gland | IL1β, IL8, TNFα |
| Raw264.7 | Mouse | Macrophage | IL1β, TNFα |
| Primary macrophage | Mouse | Macrophage | IL1β, IL6, IL10, TNFα, TGFβ1 |

### 5A. ALPK1 knockdown by siRNA in human embryonic kidney 293T (HEK293T) cells results in decreased cytokine expression

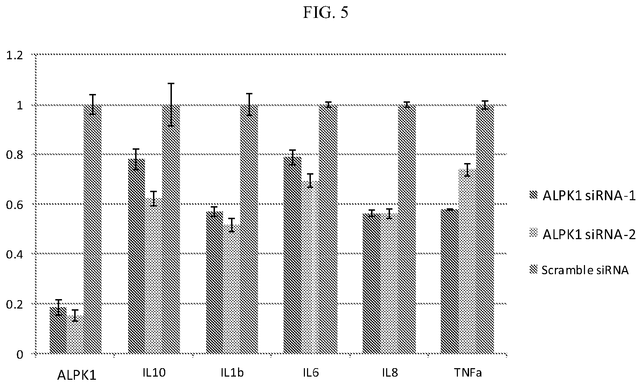

HEK293T cells were transfected with siRNA (ALPK1-siRNA-1, ALPK1-siRNA-2, Scramble siRNA) using Lipofectamine^{®} RNAiMAX Reagent (Life Technologies Corporation, Grand Island, NY). At 42 hour post transfection, HEK293T cells were harvested for RNA extraction using Trizol reagent (Life Technologies Corporation). RNA were reverse transcribed for cDNA using PrimeScript^{™} RT reagent kit (Takara Bio Inc., #RR037A) and measured by qPCR using SYBR Green I reagent on Applied Biosystems^{™} QuantStudio^{™} 7 Flex Real-Time PCR system (Life Technologies Corporation). The gene expression of each of ALPK1, IL-10, IL-1β, IL-6, IL-8, and TNF-α was normalized to GAPDH. The expression of all 5 of these pro-inflammatory cytokines was decreased by ALPK1 knockdown (Fig. 5).

### 5B. Knockdown of ALPK1 in HEK293T cells leads to decreased expression of cytokines upon S. flexneri cell lysate induction and overexpression of human and mouse ALPK1 can rescue the impaired cytokine expression, but not for the kinase dead mutant.

HEK293T cells were transfected with ALPK1-siRNA-2 or scramble siRNA using Lipofectamine^{®} RNAiMAX Reagent (Life Technologies Corporation). After 2 days, cells were transfected using LIPOFECTAMINE 3000 (LIFE) with an overexpression construct (pCDNA3.1-) containing no cDNA (Empty), or:
- ALPK1 tagged with at 1*Flag N-terminal (Flag-ALPK1)
- mouse c-terminal HA-tagged ALPK1 (mouse ALPK1)
- ALPK1 tagged with 6*His tag at N-terminal and 3*Flag at C-terminal (6*His-ALPK 1-3 *Flag)
- ALPK1tagged with 6*His tag at N-terminal (6*His- ALPK1)
- E1190A mutant of 6*His- ALPK1 (6*His- ALPK1-E mutant).

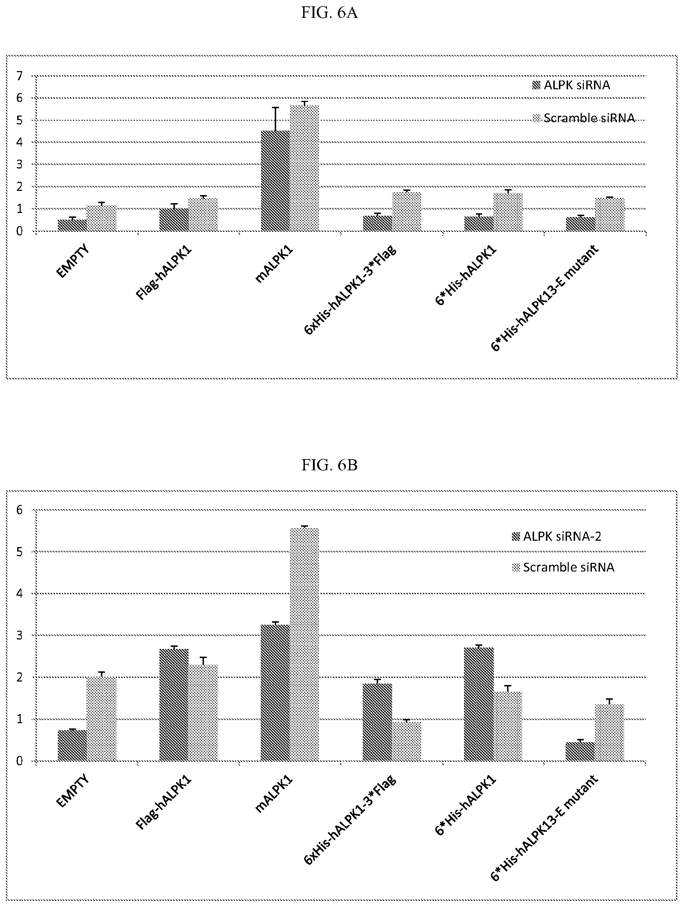

The gene expression of ALPK1 was measured both to confirm efficient knockdown of ALPK1 by siRNA and ALPK1 overexpression by overexpression vector (Fig. 6A). IL8 expression is measured (Fig. 6B) to show efficient IL8 expression by Flag- ALPK1, mouse ALPK1, 6*His- ALPK1-3*Flag, 6*His- ALPK1, but not by the 6*His- ALPK1-E mutant, suggesting the kinase activity of ALPK1 is required for ALPK1-induced IL8 expression.

### 5C. CRISPR/Cas9 knockout of ALPK1 in HEK293T cells leads to decreased cytokine expression and secretion upon S. flexneri cell lysate induction

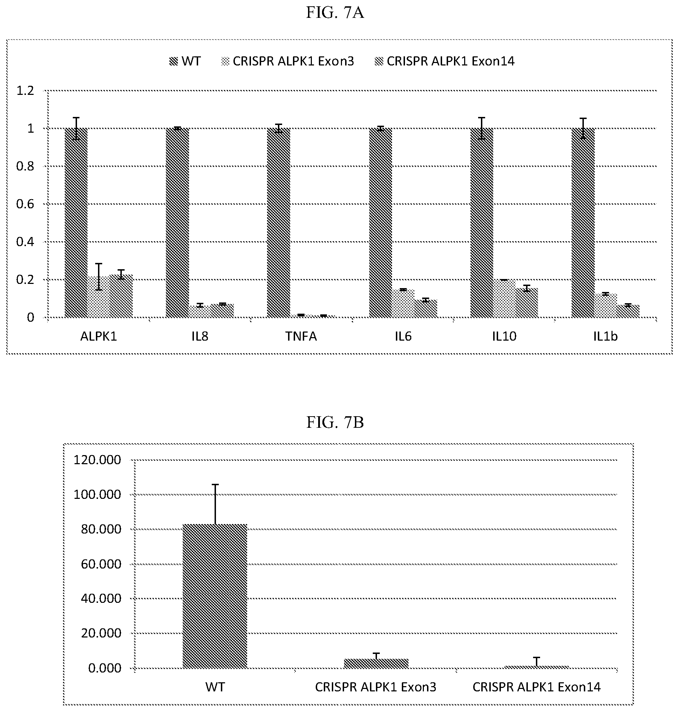

CRISPR HEK293T cells were generated by transfection of CRISPRv1.0 containing a targeting sequence to exon 3 of ALPK1 and a targeting sequence to exon 14 of ALPK1. 1 day after transfection, HEK293T cells were selected on 2.5 µg/ml puromycin for 3 days. Single colonies were expanded to generate stable cell lines. NF-κβ signaling was activated by treatment of HEK293T cells with *S. flexneri* cell lysate. After 4 hour treatment of 1% *S. flexneri* cell lysate, 293T cells were harvested for analysis of mRNA expression using Trizol reagent (LIFE). The mRNA levels of ALPK1, IL-10, IL-1β, IL-6, IL-8, and TNF-α were normalized to GAPDH expression (Fig. 7A). IL-8 secretion in the supernatant HEK293T cell culture was measured using human IL-8 ELISA kit (BD Biosciences) (Fig. 7B).

### 5D. Knockdown of ALPK1 in HEK293 cells leads to decreased expression of cytokines upon S. flexneri and S. typhimurium cell lysate induction

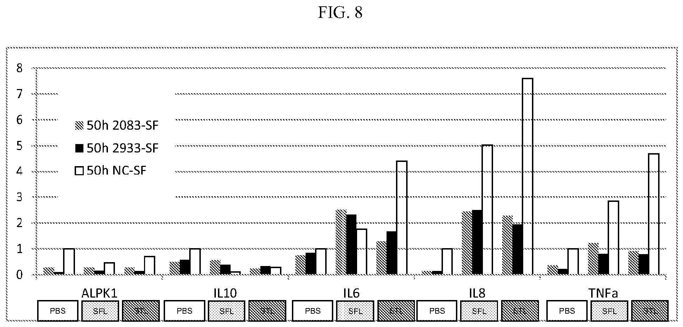

*S. flexneri* cell lysate (SFL) and *S. typhimurium* cell lysate (STL) can induce the expression of the pro-inflammatory cytokines IL6, IL8, and TNFα in HEK293 cells. Knockdown of ALPK1 by siRNA decreases the expression of all three of these cytokines (Fig. 8). siRNA were as described in 5A above.

### 5E. Knockdown of ALPK1 by shRNA in THP-1 macrophage cells leads to reduced expression of cytokines with or without LPS induction

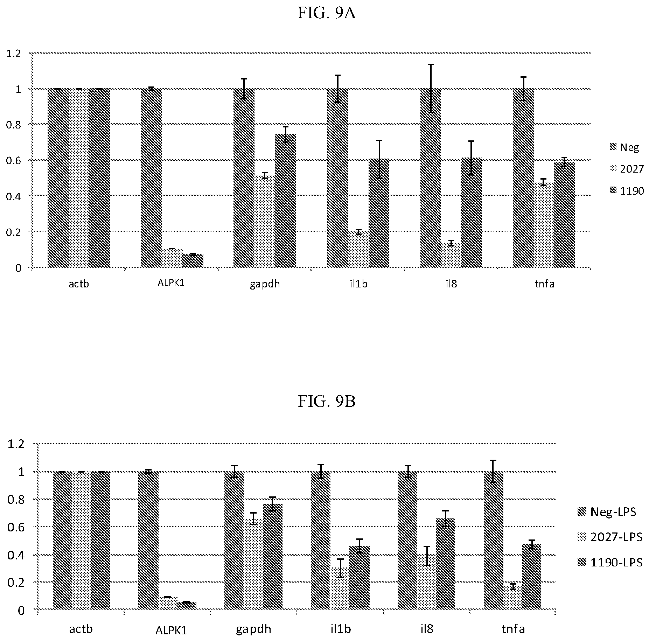

THP-1 cells were infected with Lentivirus carrying ALPK1 directed shRNA-1190, ALPK1 directed shRNA-2027, or null vector (Neg) for 5 days, then selected for using puromycin. PMA (50ng/ml) was added to induce macrophage differentiation for 2 days and LPS (10 ng/ml) was added to induce NFκβ pathway. In both LPS-stimulated cells (Fig. 9A) and unstimulated cells (Fig. 9B) the expression of IL1β, IL-8, and TNFα were reduced by shRNA knockdown of ALPK1. The expression of actin (actb) and GADPH (gadph) are also shown.

### 5F. Knockdown of ALPK1 by shRNA in THP-1 macrophage cells results in inhibition of secretion of TNFα and IL1β

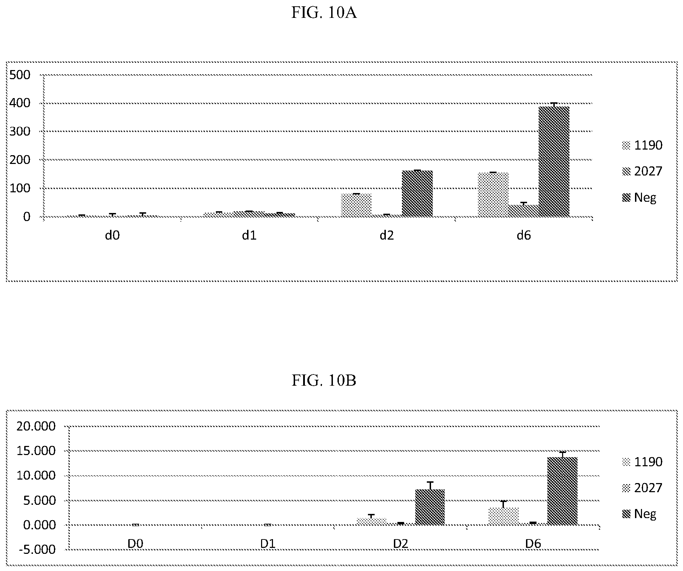

Knockdown of ALPK1 by shRNA in THP-1 macrophage leads to inhibition of secretion of TNFα (Fig. 10A) and IL1β (Fig. 10B) upon LPS (10 ng/ml) induction when THP-1 cells were induced to differentiated macrophage by PMA (50ng/ml) for 2 to 6 days.

### 5G. ALPK1 knockdown in MDA-MB-468 cells results in decreased cytokine expression

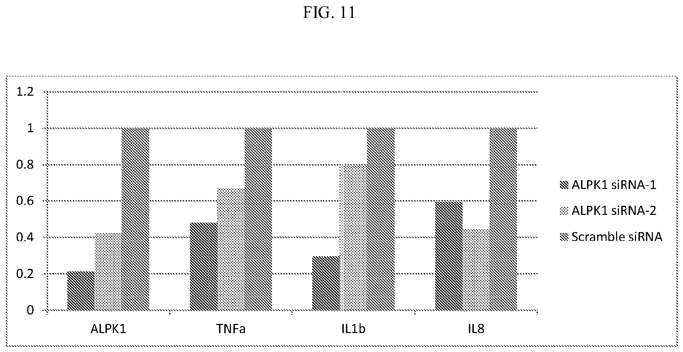

ALPK1 knockdown by siRNA in the breast cancer cell line MDA-MB-468 resulted in decreased expression of IL1β, IL8, and TNFα (Fig. 11).

### 5H. ALPK1 knockdown in RAW 264.7 cells results in decreased cytokine expression

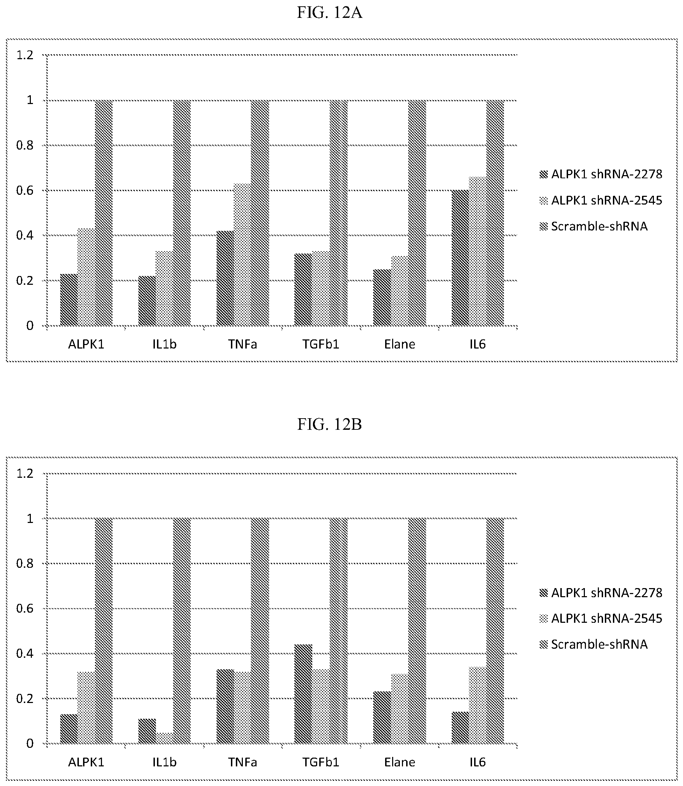

ALPK1 knockdown by shRNA in the mouse macrophage cell line RAW264.7 leads to decreased expression of cytokine with (Fig. 12A) or without (Fig. 12B) LPS (100 ng/ml) stimulation.

### 5I. Primary mouse macrophage cells from mice carrying inactivating ALPK1 mutations

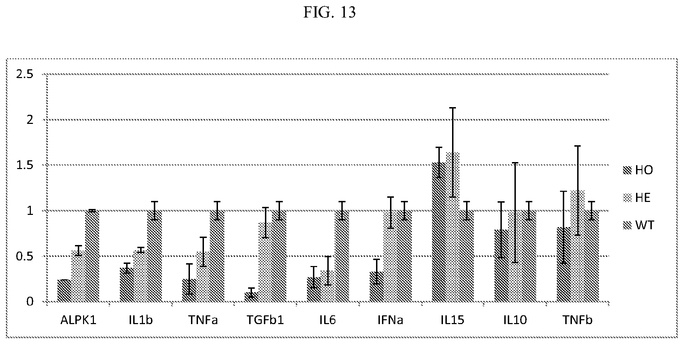

Mouse bone marrow derived macrophage cells (BMDMs) were obtained from wild type (WT) mice and the homozygous (HO) and heterozygous (HE) ALPK1 mice described in Example 1 above. Mature BMDMs were treated with 100 ng/ml LPS to induce cytokine expression. The mRNA expression of cytokines IL1β, TNFα, TGFβ1, IL6, IFNα, IL15, IL10 and TNFβ was reduced in the cells from both homozygous (HO) and heterozygous (HE) ALPK1 knockout mice (Fig. 13).

### Example 6: A small molecule kinase inhibitor, MI6, improves recovery in DSS colitis model

An ALPK1 inhibitor was identified by screening libraries of small molecules for inhibitory activity against ALPK1. Various assays were used to screen the libraries including binding assays, kinases assays, and cell-based assays for cytokine secretion. MI6 was identified as an ALPK1 inhibitor on the basis of its performance in the binding and kinase assays, as well as in at least two cell-based assays.

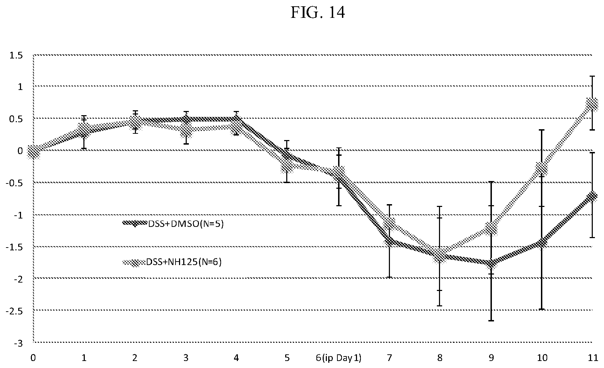

The ability of MI6 to ameliorate inflammation in the DSS-induced colitis model was evaluated as follows. 8 weeks old female wild type FVC mice were treated with 3% DSS (Sodium salt, MW 40K-50K, from Affymetrix) from day 0 to day 5. The mice were then changed to drinking water. Mice were injected intraperitoneally (IP) with either MI6 (1 mg/kg) dissolved in DMSO (n=6) or DMSO alone (n=5) daily. Animals were weighed daily. Within three days of treatment with MI6, treated animals began to show improved weight gain compared to untreated animals (Fig. 14).

### Example 7: ALPK1 mutation reduces tumor growth and metastasis in breast cancer model

In the MMTV-PyVT transgenic tumor model, polyoma virus middle T antigen (PyVT) is expressed under the mouse mammary tumor virus (MMTV) promoter, which drives mammary tissue-specific expression of PyVT. PyVT is an oncogene that activates multiple oncogenic pathways, including src and phosphatidylinositol-3-kinase, resulting in an aggressive tumor phenotype. Virgin females carrying the transgene develop multi-focal, poorly differentiated, highly invasive ductal carcinoma by 10 - 12 weeks of age, with a high incidence of lung metastases stemming from the primary mammary tumor. At 5 weeks of age females develop noninvasive focal lesions which are classified into four groups: simple, solid, cystic, and mixed (solid and cystic). Solid lesions consist of large foci with a dense mass of atypical cells in nodular sheets. Cystic lesions vary in size and complexity, and are lined by multilayered epithelium with significant amounts of clear fluid.

Metastasis of the primary tumor to distant sites remains a significant cause of death in many cancer types, highlighting the importance of a metastatic model. The MMTV-PyVT transgenic tumor model of spontaneous mammary carcinogenesis is a powerful tool for studying the mechanism associated with tumor progression and development of novel chemotherapeutics.

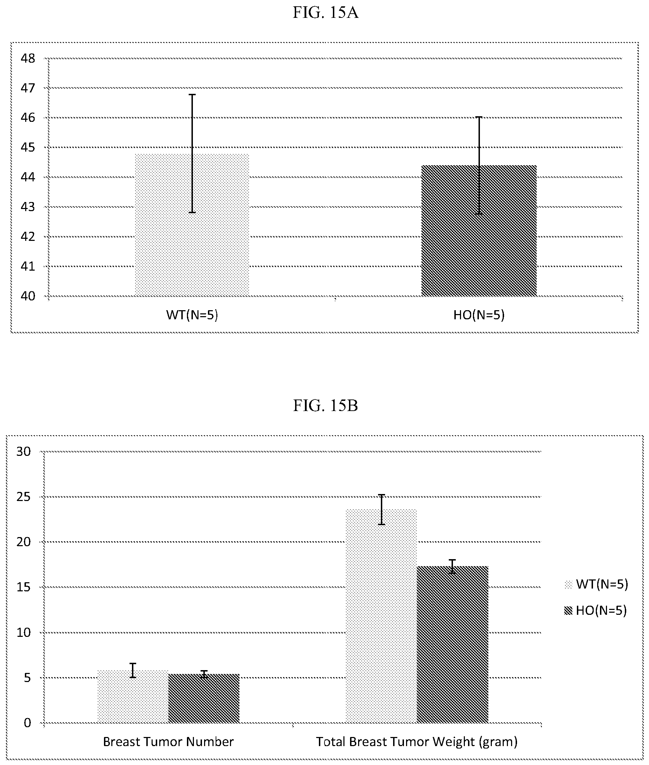

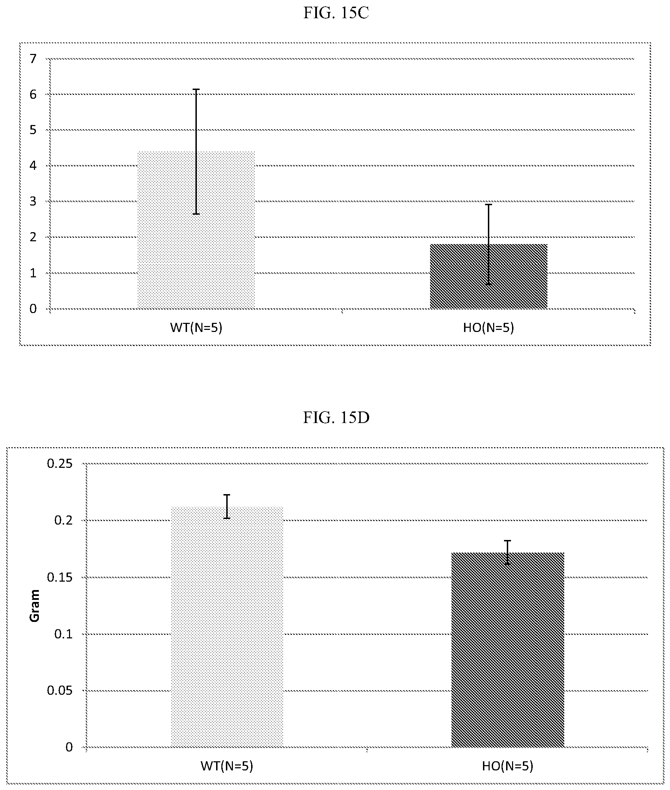

We examined the protective effects of an ALPK1 inactivating mutation in the MMTV-PyVT model. As shown in Fig. 15A-D, both the tumor load in mammary tissue (Fig. 15B), and the metastatic lung tumor load (Fig. 15D) were statistically significantly lower compared to mock MMTV-PyVT transgenic mice without the ALPK1 mutation, indicating the protective effect of this mutation.

## Claims

1. An alpha protein kinase 1 (ALPK1) inhibitor for use in a method for treating a disease or disorder caused by a bacterial infection in a subject in need of such treatment, wherein the disease or disorder is sepsis or a cytokine storm, the method comprising administering the ALPK1 inhibitor to the subject, wherein the ALPK1 inhibitor is
(i) an ALPK1 directed antibody or an anti-ALPK1-Fc fusion protein,
(ii) an ALPK1 antisense polynucleotide, or
(iii) an ALPK1-directed interfering RNA selected from the group consisting of a micro RNA (miRNA), a small interfering RNA (siRNA), and short hairpin RNA (shRNA).

2. The ALPK1 inhibitor for the use of claim 1, wherein the disease or disorder is caused by a bacteria selected from *Neisseria, Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia and Brucella.*

3. The ALPK1 inhibitor for the use of claim 1, wherein the disease or disorder is caused by a bacteria selected from *Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia and Brucella.*

4. The ALPK1 inhibitor for the use of any of the preceding claims, wherein the administration of the ALPK1 inhibitor reduces production of pro-inflammatory cytokines in the subject, and wherein the subject is human or murine.

5. The ALPK1 inhibitor for the use of any of claims 1 to 4, wherein the ALPK1 inhibitor is an ALPK1 directed antibody or an anti-ALPK1-Fc fusion protein.

6. The ALPK1 inhibitor for the use of any of claims 1 to 4, wherein the ALPK1 inhibitor is an ALPK1 antisense polynucleotide.

7. The ALPK1 inhibitor for the use of any of claims 1 to 4, wherein the ALPK1 inhibitor is an ALPK1-directed interfering RNA selected from the group consisting of a micro RNA (miRNA), a small interfering RNA (siRNA), and short hairpin RNA (shRNA).

8. The ALPK1 inhibitor for the use of any of claims 1 to 7, wherein the disease or disorder is sepsis.

9. The ALPK1 inhibitor for the use of any of claims 1 to 7, wherein the disease or disorder is a cytokine storm.

## Patentansprüche

1. Alpha-Proteinkinase-1 (ALPK1)-Inhibitor zur Verwendung in einem Verfahren zur Behandlung einer Erkrankung oder Störung, die durch eine bakterielle Infektion bei einem Subjekt verursacht wird, das einer solchen Behandlung bedarf, wobei die Erkrankung oder Störung Sepsis oder ein Zytokinsturm ist, wobei das Verfahren das Verabreichen des ALPK1-Inhibitors an das Subjekt umfasst, wobei der ALPK1-Inhibitor Folgendes ist
(i) ein gegen ALPK1 gerichteter Antikörper oder ein Anti-ALPK1-Fc-Fusionsprotein,
(ii) ein ALPK1-Antisense-Polynukleotid oder
(iii) eine gegen ALPK1 gerichtete interferierende RNA, die aus der Gruppe ausgewählt ist, die aus einer micro-RNA (miRNA), einer kurzen interferierenden RNA (small interfering RNA, siRNA) und einer kurzen Haarnadel-RNA (short hairpin RNA, shRNA) besteht.

2. ALPK1-Inhibitor zur Verwendung nach Anspruch 1, wobei die Erkrankung oder Störung durch ein Bakterium verursacht ist, das aus *Neisseria, Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia und Brucella* ausgewählt ist.

3. ALPK1-Inhibitor zur Verwendung nach Anspruch 1, wobei die Erkrankung oder Störung durch ein Bakterium verursacht ist, das aus *Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia und Brucella* ausgewählt ist.

4. ALPK1-Inhibitor zur Verwendung nach einem der vorangehenden Ansprüche, wobei die Verabreichung des ALPK1-Inhibitors eine Produktion von proinflammatorischen Zytokinen in dem Subjekt reduziert, und wobei das Subjekt human oder murin ist.

5. ALPK1-Inhibitor zur Verwendung nach einem der Ansprüche 1 bis 4, wobei der ALPK1-Inhibitor ein gegen ALPK1 gerichteter Antikörper oder ein Anti-ALPK1-Fc-Fusionsprotein ist.

6. ALPK1-Inhibitor zur Verwendung nach einem der Ansprüche 1 bis 4, wobei der ALPK1-Inhibitor ein ALPK1-Antisense-Polynukleotid ist.

7. ALPK1-Inhibitor zur Verwendung nach einem der Ansprüche 1 bis 4, wobei der ALPK1-Inhibitor eine gegen ALPK1 gerichtete interferierende RNA ist, die aus der Gruppe ausgewählt ist, die aus einer micro-RNA (miRNA), einer kurzen interferierenden RNA (siRNA) und einer kurzen Haarnadel-RNA (shRNA) besteht.

8. ALPK1-Inhibitor zur Verwendung nach einem der Ansprüche 1 bis 7, wobei die Erkrankung oder Störung Sepsis ist.

9. ALPK1-Inhibitor zur Verwendung nach einem der Ansprüche 1 bis 7, wobei die Erkrankung oder Störung ein Zytokinsturm ist.

## Revendications

1. Inhibiteur de la protéine kinase alpha 1 (ALPK1) destiné à être utilisé dans un procédé de traitement d'une maladie ou d'un trouble causé par une infection bactérienne chez un sujet nécessitant un tel traitement, dans lequel la maladie ou le trouble est une septicémie ou un orage cytokinique, le procédé comprenant l'administration de l'inhibiteur d'ALPK1 au sujet, dans lequel l'inhibiteur d'ALPK1 est
(i) un anticorps dirigé contre ALPK1 ou une protéine de fusion Fc anti-ALPK1,
(ii) un polynucléotide antisens ALPK1, ou
(iii) un ARN interférent dirigé contre ALPK1 sélectionné dans le groupe constitué d'un micro ARN (miARN), d'un petit ARN interférent (siARN) et d'un ARN en épingle à cheveux court (shARN).

2. Inhibiteur d'ALPK1 destiné à être utilisé selon la revendication 1, dans lequel la maladie ou le trouble est causé par une bactérie sélectionnée parmi *Neisseria, Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia et Brucella.*

3. Inhibiteur d'ALPK1 destiné à être utilisé selon la revendication 1, dans lequel la maladie ou le trouble est causé par une bactérie sélectionnée parmi *Escherichia, Klebsiella, Salmonella, Shigella, Vibrio, Helicobacter, Pseudomonas, Burkholderia, Haemophilus, Moraxella, Bordetella, Francisella, Pasteurella, Borrelia, Campylobacter, Yersinia, Rickettsia, Treponema, Chlamydia et Brucella.*

4. Inhibiteur d'ALPK1 destiné à être utilisé selon l'une quelconque des revendications précédentes, dans lequel l'administration de l'inhibiteur d'ALPK1 réduit la production de cytokines pro-inflammatoires chez le sujet, et dans lequel le sujet est un humain ou un murin.

5. Inhibiteur d'ALPK1 destiné à être utilisé selon l'une quelconque des revendications 1 à 4, dans lequel l'inhibiteur d'ALPK1 est un anticorps dirigé contre ALPK1 ou une protéine de fusion Fc anti-ALPK1.

6. Inhibiteur d'ALPK1 destiné à être utilisé selon l'une quelconque des revendications 1 à 4, dans lequel l'inhibiteur d'ALPK1 est un polynucléotide antisens ALPK1.

7. Inhibiteur d'ALPK1 destiné à être utilisé selon l'une quelconque des revendications 1 à 4, dans lequel l'inhibiteur d'ALPK1 est un ARN interférent dirigé contre ALPK1 sélectionné dans le groupe constitué d'un micro ARN (miARN), d'un petit ARN interférent (siARN) et d'un ARN en épingle à cheveux court (shARN).

8. Inhibiteur d'ALPK1 destiné à être utilisé selon l'une quelconque des revendications 1 à 7, dans lequel la maladie ou le trouble est la septicémie.

9. Inhibiteur d'ALPK1 destiné à être utilisé selon l'une quelconque des revendications 1 à 7, dans lequel la maladie ou le trouble est un orage cytokinique.
